# EUROPEAN PATENT APPLICATION

(11) **EP 2 135 554 A1**
(43) Date of publication of application: **23.12.2009**
(21) Application number: 08011057.0
(22) Date of filing: 18.06.2008
(51) Int. Cl.: A61B 6/04, A61B 5/055

(54) **Appliance, robot and method for performing surgical interventions**

(71) Applicant: SIEMENS AKTIENGESELLSCHAFT, 80333 München (DE)
(72) Inventor: Graupner, Harald, Dr., 91056 Erlangen (DE); Hofmann, Bernd, Dr., 91058 Erlangen (DE)

(57) **Abstract**

Appliance, robot and method for performing surgical interventions in which a patient on a surgical table can be moved by a robot between at least two positions, comprising a surgical position (SP) and at least one further position (FP), in which at least one imaging modality can be applied to the patient.

## Description

### The problem to be solved.

Performing surgical interventions (e.g. angioma or tumor surgery) requiring a combination of interventional (pre-, intra- or postoperative) fluoroscopy angiographic procedures and intra-operative (MR-) tomography is difficult and intricate to perform as the patients have to be transported to different rooms. In general the patient has to be transported from an operating room to the angiographic suite or vice versa while he is narcotized. This circumstance is prolonging the time the patient is under anesthesia which again is increasing the risk of surgical and anesthesiological complications. Furthermore, transferring the patient to different suites is also causing hygienical risks.

From a financial perspective, the prolongation of the surgical procedure results in a higher demand on staff or a reduced number of surgical procedures performed per day. Moreover, in some cases (e.g. ischemic stroke), performing tomography and angiography to come to a decision about performing further treatment is time-critical. However, patient transfer between different suites is very time consuming, resulting in delayed decision making and thus an inferior clinical outcome. As a result, reducing the patient's transport time is of major importance.

If imaging is performed in different suites, post processing, fusion and evaluation of the images is performed at different workplaces and information are not available at the "point of care" the operating theatre, in time. Furthermore, if intraoperative navigation is applied, the fluoroscopic or tomographic datasets have to be merged again by a time consuming reference process resulting in a prolonged duration of surgical intervention.

### Known prior art.

Currently, whenever possible, interventional angiography is often performed some days prior to or following surgery, requiring the patients to be anaesthetized twice afflicted with the increased anesthetic risk. If splitting the procedure is not possible, the patient is transferred to another room while being under anesthesia and the respective procedure is performed. In a few hospitals, the imaging modalities are located in adjacent rooms to the operating theatre reducing the distance for transfer or the modalities are moved to the patient.

The present inventions therefore aims at improving this sitiuation. This is achieved by an appliance, device or method according to one of the independent claims.

### Summary of the Invention.

According to the present invention, the time for patient transfer can be reduced by combining a new, space saving multi-axis, robotic system for interventional radiology and tomographic scanner or other equipment within one surgical suite. Several, e.g. two imaging modalities and the surgical position are connected by a rotating table, preferably covering an angle of 270 degrees.

In case an ultra high field MR-scanner is used, the positioning rack (positioner) which carries the surgical table has to be elongated to allow operation of the flouroscopic system outside the magnetic field (so-called 5G-line). Thereby, the center of rotation of the operating table is mounted in a distance from the scanner, allowing the removal of the surgical field away from the critical 5G isocenter by rotating the table.

The robotic stand is preferably mounted outside the critical magnetic field in a certain distance allowing access to the complete range of the surgical table. By further rotating the table, the patient can be brought into surgical position. At this position, the equipment necessary for surgery (e.g. a microscope, endoscope etc.) is not interfered by either the robotic system or the magnetic field.

In the end, e.g. three positions of the patient are defined, a surgical one, e.g. an interventional fluoroscopic one and e.g. one for tomography. In smaller operating rooms, the robotic arm can preferably be stored in a parking position ensuring enough space to bring the surgical equipment between the robotic stand and the surgical table.

Furthermore, the different positions can be adjusted to the requirements and the dimension of the surgical suite along the rotating angle. As the angiographic system is located within the surgical environment, it can be tracked by the navigational system and acquired 3-D images are directly transferred to the navigational system fused to the dataset resulting in an immediate update of the images used for navigation purposes.

The patient is anaesthetized on a stretcher either in a suite outside the operation room or directly at the surgical table. In the first case, the patient is transferred to the imaging system with the table in surgical position. In case imaging has to be performed prior to surgery, the patient is rotated either to the angiographic position or to the scanner. After imaging has been performed, the patient is rotated to surgical position and surgery is performed.

Whenever necessary, the surgical field is draped again, the patient is rotated to angiographic position or the scanner and another imaging scan is performed. After that, the patient is turned to surgical position and surgery is continued. The acquired images are post processed and fused at a workplace located adjacent or inside the operating room allowing immediate access to the surgeon. Furthermore, all data can be transferred to navigation systems.

The workflow during combination of surgery, fluoroscopy / angiography and tomography applied in one interventional procedure is optimized by combining all these diagnostic and treatment options within one surgical suite by a rotating surgical table. Imaging by performing angiography or tomography can be easily performed during surgery without transferring the patient outside the surgical suite. This results in a reduced risk for anaesthesiological complications.

As only the surgical table is rotated, there is no need to change the ventilation and monitoring system for patient transport to another room. By using a robotic fluoroscopy system a 3D image acquisition can easily be performed without using a bi-planar and more space occupying solution. Bringing the patient to the different positions is performed easily without time consuming transport. The position for surgery, angiography and imaging can be easily adapted to the available space. Data acquired by fluoroscopy / angiography are transferred immediately to a navigational system and an update is performed automatically without registration as the fluoroscopic system is integrated into the navigational system. Imaging data acquisition, post processing and evaluation is performed within the surgical suite reducing the time for decision making as the images are immediately available for the surgeon.

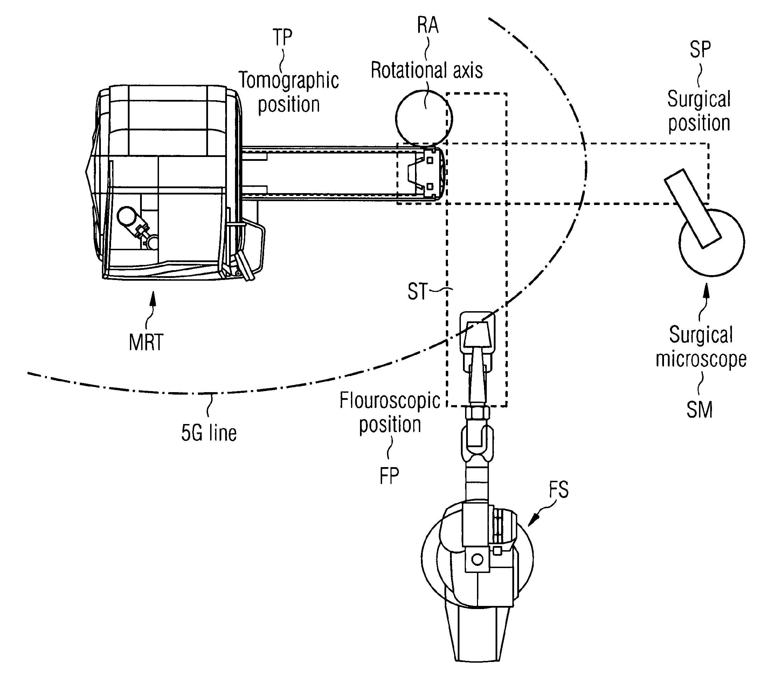

Figure 1 shows shematically a preferred embodiment of the present invention, in which a robot with at least one rotational axis is combined with a magnetic resonance tomograph, a fluoroscope and a surgical microscope.

Further solutions are available by combining tomography and angiography by using moving tables or sliding gantries within one or separate rooms. Moving either the table or the gantry of a magnet is more time consuming than just rotating it. Furthermore, hygiene aspects are more critical in the alternative solutions.

As is shown schematically in figure 1, a robot with at least one rotational axis (RA) may be combined with a magnetic resonance tomograph (MRT), a fluoroscope (FS) and a surgical microscope (SM). The robot is used to rotate the surgical tabel (ST) between various positions, comprising a tomographic position (TP), a surgical position (SP) and a fluoroscopic position (FP).

## Claims

1. Appliance for performing surgical interventions, in which a patient on a surgical table can be moved by a robot between at least two positions, comprising a surgical position and at least one further position, in which at least one imaging modality can be applied to the patient.

2. Appliance according to claim 1, in which the surgical table is carried by a positioning rack, which may be elongated to allow surgical interventions or operation of one imaging modality outside an area of perturbing influence from another imaging modality.

3. Appliance according to one of the preceding claims, where the surgical table can be rotated by a robot between at least two positions.

4. Appliance according to one of the preceding claims, where the surgical table can be translated by a robot between at least two positions.

5. Appliance according to one of the preceding claims, where the surgical table can be translated and rotated by a robot between at least two positions.

6. Robot for moving a positioning rack carrying surgical table between at least two positions, comprising a surgical position and at least one further position, in which at least one imaging modality can be applied to the patient.

7. Robot according to claim 6, where the surgical table is carried by a positioning rack, which may be elongated to allow surgical interventions or operation of one imaging modality outside an area of perturbing influence from another imaging modality.

8. Robot according to one of the preceding claims, where the surgical table can be rotated by a robot between at least two positions.

9. Robot according to one of the preceding claims, where the surgical table can be translated by a robot between at least two positions.

10. Robot according to one of the preceding claims, where the surgical table can be translated and rotated by a robot between at least two positions.

11. Method for performing surgical interventions, in which a patient on a surgical table can be moved by a robot between at least two positions, comprising a surgical position and at least one further position, in which at least one imaging modality can be applied to the patient.

12. Method according to claim 11, in which the surgical table is carried by a positioning rack, which may be elongated to allow surgical interventions or operation of one imaging modality outside an area of perturbing influence from another imaging modality.

13. Method according to one of the preceding claims, where the surgical table can be rotated by a robot between at least two positions.

14. Method according to one of the preceding claims, where the surgical table can be translated by a robot between at least two positions.

15. Method according to one of the preceding claims, where the surgical table can be translated and rotated by a robot between at least two positions.

16. Method according to one of the preceding claims, where a robotic arm of the robot can preferably be stored in a parking position ensuring enough space to bring the surgical equipment between the robotic stand and the surgical table.

17. Method according to one of the preceding claims, where an angiographic system is located within the surgical environment, which can be tracked by a navigational system and acquired 3-D images are directly transferred to the navigational system fused to the dataset resulting in an immediate update of the images used for navigation purposes.
